# EUROPEAN PATENT SPECIFICATION

(11) **EP 1 503 798 B1**
(45) Date of publication and mention of the grant of the patent: **14.12.2011**
(21) Application number: 03720286.8
(22) Date of filing: 02.05.2003
(51) Int. Cl.: A61K 47/18, A61K 38/48

(54) **STABILISED SOLID COMPOSITIONS OF MODIFIED FACTOR VII**
STABILISIERTE FESTE ZUSAMMENSETZUNGEN VON MODIFIZIERTER FAKTOR VII
COMPOSITIONS SOLIDES STABILISEES D'UN FACTEUR VII MODIFIE

(30) Priority: 03.05.2002 DK 200200677
(43) Date of publication of application: 09.02.2005
(73) Proprietor: NOVO NORDISK A/S, 2880 Bagsværd (DK)
(72) Inventor: NEDERGAARD, Hanne, DK-2100 København (DK); HANSEN, Lars, Lindgaard, DK-4621 Gadstrup (DK); KLAUSEN, Niels, Kristian, DK-2820 Gentofte (DK); KORNFELT, Troels, DK-2830 Virum (DK); FLINK, James, M., DK-2930 Klampenborg (DK)
(74) Representative: Nilsson, Karin Norvin
(86) International application number: PCT/DK2003/000287
(87) International publication number: WO 2003/092731

(56) References cited:
- WO-A-01/12653
- US-B1- 6 310 183

## Description

### FIELD OF INVENTION

The present invention relates to chemically as well as physically stable compositions comprising Modified Factor VII that can be stored, handled and used at room temperature.

### BACKGROUND OF THE INVENTION

Modified Factor VII molecules are derivatives of the blood coagulation Factor VII wherein the catalytic site is modified such that the catalytic activity of the active form, Factor VIIa, is decreased, while the ability of binding to tissue factor is maintained. Factor VII (human wild-type) has been described in U.S. Patent No. 4,784,950. Examples of Modified Factor VII molecules have been described in WO 92/15686, WO 94/27631, WO 96/12800 and WO 97/47651. Thus, in similarity to the native Factor VIIa molecule, the Modified Factor VIIa will bind to tissue factor, but conversely to native Factor VIIa, the Modified Factor VII will not activate the subsequent steps in the extrinsic pathway of coagulation. Thereby, the Modified Factor VII acts as an inhibitor of the formation of a fibrin clot. Therefore, Modified Factor VIIa molecules have been suggested in the treatment of vascular injury by blocking the production of thrombin and the subsequent deposition of fibrin (WO 97/47651).

As a protein, the Modified Factor VII molecules are susceptible to physical degradation, including denaturation and aggregation such as the formation of soluble or insoluble aggregates in the form of dimers, oligomers and polymers, or to chemical degradation, including for example, hydrolysis, deamidation and oxidation. The overall consequence is loss of activity of the Modified Factor VII molecule, formation of toxic and immunogenic degradation products, serious risk of introducing thrombosis upon injection of the degraded Modified Factor VII molecule, clogging of needles used for injections and risk of non-homogeneity. Thus safety and efficacy of medicaments comprising Modified Factor VII is directly related to the stability of Factor VII.

Modified Factor VII can today be provided in a liquid formulation, which needs to be stored frozen at - 80 °C.

Thus, compositions comprising Modified Factor VII molecules need to be stabilised. In particularly there is a need for storing and handling medicaments comprising Modified Factor VII under ambient conditions without the requirement of a freezer and wherein the compositions can be stored for a prolonged time such as for at least 6 months before use.

One approach of stabilising a protein relates to removal of water from the protein, e.g. such as providing the protein in the form of a lyophilised cake, the final matter obtained in a freeze-drying process. However, the freeze-drying process itself is also harmful to proteins; during freeze-drying, the protein solution is first cooled until adequately frozen and bulk water in the protein solution will form ice at this stage. The protein is hereby prone to freeze-induced stress resulting in deformation and precipitation. In the next step, the so-called primary drying stage, the ice sublimes and in the secondary drying stage, adsorbed or bound water is removed under elevated temperatures. During this water removal, the proteins may loose their proper conformation that is provided mainly through hydrogen bonding.

Therefore, to preserve protein conformation, activity and stability during freeze-drying, the protein solution needs to be supplemented with sufficient amounts of proper excipients with cryoprotectant and/or lyoprotectant properties so as to protect the protein from freeze-induced stress and/or stress during removal of water, respectively.

When providing a lyophilised product an essential feature relates to the properties of the lyophilised cake. It needs to have good properties as to its form and structure, i.e. it should not collapse in that such collapsed cakes can be hard or even impossible to dissolve (reconstitute) before use. Conversely, the physical structure of the lyophilised cake may not be to loose and soft. Therefore, one or more so-called bulking agents are added to the protein solution before freeze-drying. Bulking agents are agents which provide good lyophilised cake properties and which help the protein overcoming various stresses associated with the lyophilisation process, for example shear/freezing. Furthermore, the bulking agents may help in forming pharmaceutically elegant and nice-looking products, protecting the protein during freeze-drying as well as during subsequent storage.

When developing a stable medicament comprising a Modified Factor VII appropriate for being parental administered and being stored at ambient conditions, suitable excipients ought to be added and their levels carefully adjusted in order to provide a product that also is approximately isotonic with plasma and has a pH in a physiologically suitable range for injection or infusion. The choice of agents capable of modifying the tonicity is crucial in that most tonicity modifiers in the form of salts make the freeze-drying process difficult.

Thus, it is an objective of the present invention to provide stable compositions of Modified Factor VII, substantially without the presence of degradation products and without decreased activity of the Modified Factor VII, even after prolonged storage at ambient conditions. Furthermore, it is an essential objective that the stable compositions are suitable for parental administration and thus have a physiologically proper tonicity and pH range so as not to cause any inconvenience for the patient.

### SUMMARY OF THE INVENTION

It has been found by the present investigators that Modified Factor VII can be provided in a composition that is sufficient stable so as to allow for storage at 25 °C for about at least 12 to 18 months.

Accordingly, the present invention relates in a first aspect to stabilised compositions comprising:
i) Modified Factor VII;
ii) an agent suitable for keeping the pH of said composition in the range of 4 to 7 when said composition is dissolved in water; and further ingredients as in the claims and
iii) a moisture content of at the most 3%.

In a further aspect, the invention relates to a method of preparing a stable Modified Factor VII comprising the steps of:
i) providing said Modified Factor VII in a solution with pH in the range of 4 to 7.
ii) processing said solution so as to obtain a solid composition with a moisture content of at the most 3 % w/w.

As mentioned, stabilised Modified Factor VII is requested so as to minimise the risk of adverse events and to improve safety and efficacy when administering Modified Factor VII for therapeutic purposes. Therefore, a still further aspect of the invention relates to the use of a Modified Factor VII for the preparation of a medicament for preventing blood clotting and/or preventing tissue factor mediated reactions, said medicament comprising a composition comprising;
i) Modified Factor VII;
ii) an agent suitable for keeping the pH of said composition in the range of 4 to 7 when said composition is dissolved in water, and further ingredients as in the claims and
iii) a moisture content of at the most 3%.

Finally, the invention relates to administering said stabilised Modified Factor VII to a patient for preventing blood clotting and/or preventing tissue factor mediated reactions, comprising administering to a subject in need thereof, an effective amount of a composition comprising;
i) Modified Factor VII;
ii) an agent suitable for keeping the pH of said composition in the range of 4 to 7 when said composition is dissolved in water; and further ingredients as in the claims and
iii) a moisture content of at the most 3%.

### DETAILED DESCIPTION OF THE INVENTION

The present invention relates to storage-stable compositions comprising Modified Factor VII. The compositions can be stored at various temperatures including ambient temperatures for an extended period of time without causing substantial degradation of the Modified Factor VII.

The present invention is based on the discovery of storage-stable formulations of Modified Factor VII that are stable for at least about 12 to 18 months upon storage at 25 °C in the dark. The present Invention also encompasses compositions comprising Modified Factor VII that are stable even for longer periods of storage, such as 32 months. Thus, the present invention makes it possible to store such compositions at room temperature without increasing the risk of adverse events to the patient administering such compositions. Advantageously, the improved storage-stability will also result in reduced cost in that no special cooled conditions are required upon storage, further resulting in more convenient handling of the composition at the user.

The term Modified Factor VII is denoted to mean any Factor VII protein, which is modified so as to decrease the catalytic activity of Factor VIIa, while still being able of binding to tissue factor. As a result, the Modified Factor VII molecules will compete with native Factor VII and/or VIIa for binding to tissue factor, thereby inhibiting the activation of the subsequent Factors, X and IX, of the coagulation sequence. The modification may, for example, be located within the catalytic site.

The term "Modified factor VII" encompasses, without limitation, polypeptides in which the factor VIIa biological activity has been substantially reduced relative to the activity of wild-type human factor VIIa (as disclosed in US Patent No. 4,784,950).
These polypeptides include, without limitation, factor VII or factor VIIa that has been chemically modified and factor VII variants into which one or more specific amino acid sequence alterations have been introduced that modify or disrupt the bioactivity of the polypeptide. The term "Modified Factor VII" further encompasses, without limitation, polypeptides that have truncated amino acid sequences relative to human factor VII (i.e., factor VII fragments), and/or modified N-terminal end including N-terminal amino acid deletions or additions, and/or posttranslational modifications. The Modified Factor VII binds to tissue factor with high affinity and specificity but do not initiate blood coagulation.

As used herein, Modified Factor VII can be in the form of the zymogen (i.e., a single-chain molecule) or can be cleaved at its activation site. Thus, the term "Modified Factor VII" is intended to mean a Modified Factor VII that, upon activation to Modified Factor VIIa, as well as the Modified Factor VIIa molecules that are capable of binding tissue factor, capable of competing with authentic Factor VII and/or Factor VIIa for binding to tissue factor in the coagulation cascade thereby inhibiting the activation of Factor IX to IXa and Factor X to Xa. Such competition may readily be determined by means of, e.g., a competition clotting assay, a competition FIXa or FXa generation assay, or a competition binding assay, using, e.g., a cell line having cell-surface tissue factor, such as the human bladder carcinoma cell line J82 (Sakai et al. J. Biol. Chem. 264: 9980-9988 (1989), incorporated by reference herein.), for example, as described in the present specification (see "Assay" section, below),

In one embodiment of the invention, Modified Factor VII encompass those polypeptides that exhibit at least about 25%, at least about 50%, at least about 75%, at least about 80%, at least about 90%, at least about 100%, at least about 110%, or at least about 120% of the specific TF-binding affinity of wild-type factor VIIa, when tested in one or more of the TF binding assays as described in the present specification. In a preferred embodiment, the TF antagonists exhibit at least about 75% of the binding affinity of wild-type factor VIIa. The term "TF binding activity" as used herein means the ability of a FVlla polypeptide or TF antagonist to inhibit the binding of recombinant human 125I-FVIIa to cell surface human TF. The TF binding activity may be measured, for example, as described in Assay 3 (of the present specification).
In another embodiment, Modified Factor VII encompass those polypeptides that exhibit less than about 25%, more preferably less than about 10%, or 5%, or 3%, or 2%, and most preferably less than about 1 % of the specific activity of wild-type factor VIIa, when tested in one or more of a clotting assay, FIXa or FXa generation assay, amidolysis or proteolysis assay as described in Assays 1-2 and 4-7 of the present specification.

The term "catalytic site" or "active site", when used herein with reference to FVIIa, refer to the catalytic and zymogen substrate binding site, including the "S₁" site of FVlla as that term is defined by Schecter, I. and Berger, A., (1967) Biochem. Biophys. Res. Commun. 7:157-162.

The catalytic site of human and bovine Factor VII proteins comprises the amino acids Ser344, Asp242, and His193 (subscript numbering indicating position in the sequence) that are forming a so-called catalytic "triad". The catalytic sites in Factor VII from other mammalian species may be determined using presently available techniques including, among others, protein isolation and amino acid sequence analysis. Catalytic sites may also be determined by aligning a sequence with the sequence of other serine proteases, particularly chymotrypsin, whose active site has been previously determined (Sigler et al., J. Mol. Biol., 35:143-164 (1968), incorporated herein by reference), and therefrom determining from said alignment the analogous active site residues. Thus, as used herein, Modified Factor VII encompasses Factor VII polypeptides derived from any mammalian species.

The modification of the catalytic activity of Factor VIIa can be effected by a number of ways, including, without limitation, chemical derivatization, enzymatic reactions or by substituting, inserting or deleting amino acids.

In one embodiment the catalytic activity of factor VIIa is inhibited by chemical derivatization of the catalytic site, or triad. Derivatization may be accomplished by reacting factor VII with an irreversible inhibitor such as an organophosphor compound, a sulfonyl fluoride, a peptide halomethyl ketone or an azapeptide, or by acylation, by non-limiting example. Inhibitors include, without limitation, peptide chloromethylketones or peptidyl cloromethanes; azapeptides; acylating agents such as various guanidinobenzoate derivatives and 3-alkoxy-4-chloroisocoumarins; sulphonyl fluorides such as phenylmethylsulphonylfluoride (PMSF); diisopropylfluorophosphate (DFP); tosylpropylchloromethyl ketone (TPCK); tosylysylchloromethyl ketone (TLCK); nitrophenylsulphonates; heterocyclic protease inhibitors such as isocoumarines, and coumarins.

Preferred peptide halomethylketones include Phe-Phe-Arg chloromethylketone (FFR-cmk), D-Phe-Phe-Arg chloromethylketone (D-FFR-cmk), Phe-Pro-Arg chloromethylketone (FPR-cmk), D-Phe-Pro-Arg chloromethylketone (D-FPR-cmk) (see U.S. Patent No. 4,318,904 incorporated herein by reference), L-Glu-Gly-Arg chloromethylketone (EGR-cmk) and D-Glu-Gly-Arg chloromethylketone (D-EGR-cmk), Dansyl-Phe-Phe-Arg chloromethyl ketone, Dansyl-D-Phe-Phe-Arg chloromethylketone, Dansyl-Phe-Pro-Arg chloromethylketone, Dansyl-D-Phe-Pro-Arg chloromethylketone, Dansyl-L-Glu-Gly-Arg chloromethylketone and Dansyl-D-Glu-Gly-Arg chloromethylketone.

In the embodiment where the Modified Factor VII molecule is in the activated form, it is possible to modify FVIIa by reaction with a serine protease inhibitor. In one embodiment, the protease inhibitor is an organophosphor compound, a sulfanyl fluoride, a peptide halomethyl ketone, or an azapeptide. In one embodiment, the protease inhibitor is a peptide halomethyl ketone selected from Phe-Phe-Arg chloromethylketone (FFR-cmk), D-Phe-Phe-Arg chloromethylketone (D-FFR-cmk), Phe-Pro-Arg chloromethylketone (FPR-cmk), D-Phe-Pro-Arg chloromethylketone (D-FPR-cmk), L-Glu-Gly-Arg chloromethylketone (EGR-cmk) and D-Glu-Gly-Arg chloromethylketone (D-EGR-cmk), Dansyl-Phe-Phe-Arg chloromethyl ketone, Dansyl-D-Phe-Phe-Arg chloromethylketone, Dansyl-Phe-Pro-Arg chloromethylketone, Dansyl-D-Phe-Pro-Arg chloromethylketone, Dansyl-L-Glu-Gly-Arg chloromethylketone and Dansyl-D-Glu-Gly-Arg chloromethylketone. In one embodiment, the protease inhibitor is a peptide halomethyl ketone selected from Dansyl-L-Phe-Pro-Arg chloromethyl ketone, Dansyl-L-Glu-Gly-Arg chloromethyl ketone, Dansyl-L-Phe-Phe-Arg chloromethyl ketone and L-Phe-Phe-Arg chloromethylketone, Dansyl-D-Phe-Pro-Arg chloromethyl ketone, Dansyl-D-Glu-Gly-Arg chloromethyl ketone, Dansyl-D-Phe-Phe-Arg chloromethyl ketone and D-Phe-Phe-Arg chloromethylketone. In one embodiment, the protease inhibitor is D-Phe-Phe-Arg chloromethylketone (FFR-FVIIa).

It is to be understood that when the designation "D" immediately precedes a letter abreviation for an amino acid as shown above, that amino acid is the non-natural d-enantiomer

In further embodiments the catalytic activity of Factor VIIa is inhibited by substituting, inserting or deleting amino acids.
In one series of embodiments, one or more amino acid substitutions are made in the amino acid sequence of the factor VII catalytic triad, defined herein as the regions which contain the amino acids which contribute to the factor VIIa catalytic site. The substitutions, insertions or deletions in the catalytic triad are generally at or adjacent to the amino acids which form the catalytic site. In the human and bovine factor VII proteins, the amino acids which form a catalytic "triad" are Ser344, Asp242, and His193 (subscript numbering indicating position in human wild type factor VII). The catalytic sites in factor VII from other mammalian species may be determined using presently available techniques including, among others, protein isolation and amino acid sequence analysis. Catalytic sites may also be determined by aligning a sequence with the sequence of other serine proteases, particularly chymotrypsin, whose active site has been previously determined (Sigler et al., J. Mol. Biol., 35:143-164 (1968), incorporated herein by reference), and therefrom determining from said alignment the analogous active site residues.

The amino acid substitutions, insertions or deletions are made so as to prevent or otherwise inhibit activation by the factor VIIa of factors X and/or IX. The factor VII so modified should, however, also retain the ability to compete with authentic factor VII and/or factor VIIa for binding to tissue factor in the coagulation cascade. Such competition may readily be determined by means of, e.g., a clotting assay or a competition binding assay as described in the present specification, using, e.g., a cell line having cell-surface tissue factor, such as the human bladder carcinoma cell line J82 (Sakai et al. J. Biol. Chem. 264: 9980-9988 (1989)).

Within the present invention, it is preferred to change only a single amino acid, thereby minimizing the likelihood of increasing the antigenicity of the molecule or inhibiting its ability to bind tissue factor. However, two or more amino acid changes (substitutions, additions or deletions) may be made and combinations of substitution(s), addition(s) and deletion(s) may also be made. The amino acids that form the catalytic site in Factor VII, such as Ser344, Asp242, and His193 in human and bovine Factor VII, may either be substituted or deleted. In a preferred embodiment for human and bovine Factor VII, Ser344 is preferably substituted with Ala, but Gly, Met, Thr or other amino acids can also be substituted. It is preferred to replace Asp with Glu and to replace His with Lys or Arg. In general, substitutions are chosen to disrupt the tertiary protein structure as little as possible. The model of Dayhoff et al. (in Atlas of Protein Structure 1978, Nat. Biomed. Res. Found., Washington, D.C.), incorporated herein by reference, may be used as a guide in selecting other amino acid substitutions. One may introduce residue alterations as described above in the catalytic site of appropriate Factor VII sequence of human, bovine or other species and test the resulting protein for a desired level of inhibition of catalytic activity and resulting anticoagulant activity as described herein.

In preferred embodiments of human and bovine Factor VII, the active site residue Ser344 is modified, replaced with Gly, Met, Thr, or more preferably, Ala. Such substitution could be made separately or in combination with substitution(s) at other sites in the catalytic triad, which includes His193 and Asp242.

Non-limiting examples of Modified Factor VII having substantially reduced biological activity relative to wild-type factor VII include R152E-FVIIa (Wildgoose et al., Biochem 29:3413-3420, 1990), S344A-FVIIa (Kazama et al., J. Biol. Chem. 270:66-72, 1995), FFR-FVlla (Holst et al., Eur. J. Vasc. Endovasc. Surg. 15:515-520, 1998), and factor Vlla lacking the Gla domain, (Nicolaisen et al., FEBS Letts. 317:245-249, 1993). Non-limiting examples also include human FVIIa, which has the lysine residue in position 341 replaced by another amino acid residue; human FVlla, which has the serine residue in position 344 replaced by another amino acid residue; human FVlla, which has the aspartic acid residue in position 242 replaced by another amino acid residue; human FVlla, which has the histidine residue in position 193 replaced by another amino acid residue; FVII-(K341A); FVII-(S344A); FVII-(D242A); FVII-(H193A); Phe-Phe-Arg-FVII (FFR-FVII), D-Phe-Phe-Arg-FVII (D-FFR-FVII), Phe-Pro-Arg-FVII (FPR-FVII), D-Phe-Pro-Arg-FVII (D-FPR-FVII), L-Glu-Gly-Arg-FVII (EGR-FVII) and D-Glu-Gly-Arg-FVII (D-EGR-FVII), Dansyl-Phe-Phe-Arg-FVII, Dansyl-D-Phe-Phe-Arg-FVII, Dansyl-Phe-Pro-Arg-FVII, Dansyl-D-Phe-Pro-Arg-FVII, Dansyl-L-Glu-Gly-Arg-FVII and Dansyl-D-Glu-Gly-Arg-FVII.. Non-limiting examples of chemically modified factor VII polypeptides and sequence variants are described, e.g., in U.S. Patent No. 5,997,864.

Moreover, the term "Modified Factor VII" relates to Modified Factor VII molecules that are derived from animals, such as humans or produced by recombinant and/or synthetic means.

In one embodiment, the Modified Factor VII molecule is Factor VIIa, preferably bovine or human Factor VII, which is modified by the protease inhibitor D-Phe-Phe-Arg chloromethylketone.

In one embodiment, the Modified Factor VII molecule is human wild-type Factor VIIa modified by the protease inhibitor D-Phe-Phe-Arg chloromethylketone. In that embodiment the Modified Factor VII molecule is abbreviated FFR-FVIIa.

As used herein, FFR-FVIIa concentration is conveniently expressed as mg/ml or as U/ml, with 1 mg/ml approximately equivalent to 20 U/ml.

The modified factor VII polypeptide may be present in a concentration of from about 0.1 mg/ml to about 10.0 mg/ml, such as from about 0.5 to about 8.0 mg/ml, from about 0.5 to about 5.0 mg/ml, or from about 1.0 mg/ml to about 5.0 mg/ml.

As used herein, "stabilising" encompasses minimising the formation of aggregates (insoluble and/or soluble) and/or chemical degradation as well as providing maintenance of pH and proper conformation of the protein during storage or production of the compositions so that substantial retention of biological activity and protein stability is maintained. Moreover, stabilising also encompasses lyoprotection and cryoprotection of the protein during production of the compositions at freeze-drying conditions.

The term "structural stabilisation" or "structural stability" encompasses the ability to form a lyophilised plug or cake with good properties and looks, e.g. such that it does not collapse and is readily dissolved before use.

A storage-stable product is a product that is stabilised upon storage at temperatures between 5°C - 50°C and remains within pre-selected product specifications for a predetermined time period - often several months.

Physical stability relates to the formation (or lack thereof) of insoluble and/or soluble aggregates in the form of dimeric, oligomeric and polymeric forms of Modified Factor VII as well as any structural deformation and denaturation of the molecule.

Chemical stability relates to the formation (or lack thereof) of any chemical change in the Modified Factor VII upon storage in dissolved or solid state at accelerated conditions. By non-limiting example are hydrolysis, deamidation and oxidation. In particularly, the sulphur-containing amino acids are prone to oxidation with the formation of the corresponding sulphoxides.

The term "cryoprotectants" as used herein generally includes agents, which provide stability to the protein from freezing-induced stresses. Non-limiting examples of cryoprotectants include polyols such as, for example, mannitol, and include saccharides such as, for example, sucrose, as well as including surfactants such as, for example, polysorbate, poloxamer or polyethylene glycol, and the like. Cryoprotectants also contribute to the tonicity of the formulations.

The term "lyoprotectant" as used herein includes agents that provide stability to the protein during water removal upon the drying process of the lyophilisation process, such as, for example by maintaining the proper conformation of the protein. Non-limiting examples of lyoprotectants include saccharides, in particularly di- or trisaccharides. Cryoprotectants may also have lyoprotectant effects.

The term "agent suitable for keeping the pH in the range of 4 to 7" encompasses those agents that maintain the solution pH in an acceptable range between 4.0 to 7.0. Typical non-limiting examples of agents capable of keeping the pH within a range of 4 to 7 are citrate (sodium or potassium), acetate (ammonium, sodium or calcium), histidine (L-histidine), malate, phosphate (sodium or potassium), tartaric acid, succinic acid, MES, HEPES, imidazol, TRIS, lactate, glutamate and glycylglycine.

The term "lyophilised cake" as used herein encompasses the solid composition obtained upon processing a dissolved or at least a partly dissolved composition under conditions involving at least one step of cooling said dissolved/partly dissolved composition to ice followed by at least one step of vacuum drying.

The term "lyophilization" and "freeze-drying" encompasses a process during which liquid is removed from a dissolved or at least partly dissolved composition under conditions involving at least one step of cooling the dissolved or partly dissolved solution to ice followed by vacuum drying. Lyophilization, or freeze-drying, is the most common process for making solid protein pharmaceuticals. The process consists of two major steps: freezing of a protein solution, and drying of the frozen solid under vacuum. The drying step is further divided into two phases: primary and secondary drying. The primary drying removes the frozen water (sublimation of ice) and the secondary drying removes the non-frozen "bound" water (desorption of water). More detailed analysis of each lyophilization step is provided in, e.g., Wang et al, International Journal of Pharmaceutics 203 (2000): 1-60 (see section 4, page 16 ff.).

Typically, a composition is freeze-dried by filling into vials, freezing on the shelves of the freeze-dryer, after which a vacuum is established and the shelves heated to implement primary drying (or sublimation of ice). Thereafter, secondary drying (or desorption of sorbed water) takes place at a higher temperature until the completion of the process, i.e., where the composition contains a sufficiently low content of moisture (water). Methods for freeze-drying are generally known in the art, see, for example, Wang et al, International Journal of Pharmaceutics 203 (2000): 1-60.
It is within the ordinary skill of the practitioner to optimize the freeze-drying conditions in regard of temperature(s), time(s) at each temperature, and also pressure that is to be used during the process for a specific composition.

The term "moisture content" is meant to encompass water associated with the product, including, without limitation, water in adsorbed form, such as unfrozen water entrapped in or adsorbed to the frozen solute phase and/or associated with the amorphous phase or adsorbed to the crystalline solid. The term "water content" is used interchangeably with "moisture content". The desired residual moisture level (moisture content) in a function of the duration and the temperature of the secondary drying step. Several methods for determining the residual moisture content during lyophilization are known in the art; for example, an electronic hygrometer or a residual gas analyser may be used. Moisture contents of freeze-dried formulations can be determined by several methods known in the art, such as, for example, loss-on-drying, Karl Fischer titration, thermal gravimetric analysis (TGA), gas chromatography (GC), or near IR (see, e.g. Wang et al, international Journal of Pharmaceutics 203 (2000): 1-60). Methods for determining water contents (moisture contents) are also described in both the European and U.S. Pharmacopoeias. For example, determination of water content can be performed by Karl Fischer coulometric titration as described in the U.S. Pharmacopoeia (USP <921, Ic>) or the European Phamacopoeia (EP <2.5.32>). In brief, the method is as follows:
Determination of water content by coulometric titration: The Karl Fischer reaction is used in the coulometric determination of water based upon the quantitative reaction of water with sulphur dioxide and iodine in an anhydrous medium. Iodine is produced electrochemically in the reaction cell by oxidation of iodide. The iodine produced at the anode reacts immediately with the water and the sulphur dioxide contained in the reaction cell. The amount of water in the substance is directly proportional to the quantity of electricity up until the titration end-point. When all of the water in the cell has been consumed, the end-point is reached and thus an excess of iodine appears which is detected electrometrically, thus indicating the end-point. The percentage water content present in the substance is then calculated.

Moisture content may be defined in terms of the weight of the sample in the vial at the time of analysis (i.e. solids plus the water present- called wet weight basis) or it may be defined in terms where it is corrected for the measured water in the sample (i.e. dry weight basis). In case of freeze-dried products with low moisture contents the two measurements (wet weight basis vs. dry weight basis) yield very similar results. As used herein, moisture contents are defined in terms of the solids plus the water present (i.e., wet weight basis).

The term "bulking agent" generally includes agents, which provide good lyophilised cake properties, which form a pharmaceutically elegant product, which help the protein overcome various stresses, shear/freezing for example, associated with lyophilisation processes, and which help to maintain protein activity levels during the freeze-drying process and subsequent storage. Non-limiting examples of bulking agents include mannitol, glycine, sucrose, lactose. These agents may also contribute to the tonicity of the formulations.

The term "tonicity modifier" encompasses any agent capable of contributing to the osmolalily of the solution, thereby adjusting the tonicity of the composition such that upon dissolving the composition at the time of use, the composition has a tonicity within the physiological range of the blood, peritoneal fluid or other relevant body fluids. Obviously, the tonicity may also depend on whether the reconstitution solution comprises tonicity-modifying agents.

The term "surfactants" generally include those agents, which protect the protein from air/solution interface-induced stresses and solution/surface induced-stresses. For example surfactants may protect the protein from aggregation. Suitable surfactants may include, without limitation, polysorbate such as Tween 20, Tween 80, or poloxamer such as poloxamer 188 or 407, and other ethylene/polypropylene block polymers or polyethyleneglycol (PEG) such as PEG8000. Preferred detergents are poloxamers, e.g. Poloxamer 188, Poloxamer 407; alkyl ethers e.g. Cremophor A25, Sympatens ALM/230; and polysorbates/Tweens, e.g. Polysorbate 20, Polysorbate 80. More preferred are Poloxamers, e.g. Poloxamer 188, and Tweens, e.g. Tween 20 and Tween 80.

The term "theoretical content" relates to the amount of Modified Factor VII added to a composition at the time of preparation. The concentration given herein (mg/ml) refer to either the concentration in the solution of Modified Factor VII before Freeze-drying or is referred as % w/w (weight/weight), which then relates to the concentration in the lyophilised cake.

As used herein, amounts specified are understood to be ± about 10%; thus about 50 mM includes 50 mM ± 5mM, 4% includes 4% ± 0.4%, etc.

As stated above, the present invention provides methods and compositions for stabilising Modified Factor VII proteins thereby allowing long-term storage without causing increased risk and inconvenience to the user.

The present investigators have found that a number of crucial parameters need to be adjusted in stabilising modified Factor VII. As a first parameter relates, at least in part, the moisture content, e.g. water. The moisture content should be limited. As a further essential parameter, the composition should include an agent suitable for keeping the pH within of pH 4 to 7.

Thus, in a first aspect, the invention relates to a composition comprising:
i) Modified Factor VII;
ii) an agent suitable for keeping the pH of said composition in the range of 4 to 7 when said composition is dissolved in water; and
iii) a moisture content of at the most 3%.

The Modified Factor VII that can be formulated in accordance with the present invention is described above. In suitable embodiments of the invention, the Modified Factor VII is selected from the group consisting of dansyl-EGR-FVIIa, dansyl-EGR-FVII, FFR-FVlla, FFR-FVII, PPA-FVIIa, PPA-FVII, Ser344-FVIIa and Ser344-FVII. Preferably, the Modified Factor VII is FFR-FVIIa or FFR-FVII.

As stated the pH should be kept in the pH range within 4 to 7 when dissolved in water. Advantageously, this pH range is also within the desired physiological range, thereby causing no harm to the user upon administering the composition by parental means. Preferably the solution pH is 5.0 to 7.0, 5.5 to 7.0 or 5.8 to 6.8 such as close to a pH of 6.0 and 6.5.

Some of the suitable agents for keeping the pH within the desired range need to be added in limited amounts in order to achieve the desired pH. For example, when the agent is glycylglycine, its content needs to be adjusted.

Thus, in still further interesting embodiments, wherein the agent suitable for keeping the pH of said composition in the range of 4 to 7 is glycylglycine, it is present in an amount of at the most 7 mg/ml. Accordingly, in suitable embodiments thereof, the agent suitable for keeping the pH in the range of 4 to7 is selected from the group consisting of citrate, histidine, malate, phosphate, tartaric acid, succinic acid, MES, HEPES, imidazol, TRIS, lactate, glutamate and glycylglycine, with the proviso that when said agent is glycylglycine, it is present in an amount of at the most 7 mg/ml.

In further embodiments related hereto, the amount of glycylglycine is even more restricted, such that the amount of glycylglycine is of at the most about 4 mg/ml, preferably of at the most about 3 mg/ml, most preferably of at the most about 2 mg/ml.

Furthermore, the suitable agent for keeping the pH in the range of 4 to 7 may also be a mixture of at least two such listed agents, wherein the mixture is able to provide a pH value in the specified range. The concentration of the suitable agents is In the range of from about 1 mM to 100 mM; from 1 mM to about 50 mM; from about 1 mM to about 25 mM; from about 2 mM to about 20 mM; or about 10 mM.

As stated above, the moisture content should be limited. Thus, in suitable embodiments of the invention, the moisture content is at the most 2% w/w, most preferably at the most about 1% w/w.

Typically, Modified Factor VII proteins, when provided in bulk, are in liquid form. Thus, further processing of the bulk proteins for the manufacturing of compositions requires the steps of adding suitable excipients and removing the liquid from the bulk, said addition of excipients may be carried out before or after removing the liquid. One such mean for removing liquid from a protein relates to freeze-drying. Therefore, in a preferred embodiment of the present invention, the composition is the form of a lyophilised cake.

Thus, in suitable embodiments according to the invention, the composition further comprises cryoprotectants, lyoprotectants and/or bulking agents. In one embodiment thereof, the composition further comprises a sugar alcohol selected from the group consisting of mannitol, sorbitol and xylitol. In another embodiment thereof, the composition further comprises a saccharide selected from the group consisting of sucrose, dextrose, lactose, maltose, trehalose, cyclodextrins, maltodextrins and dextrans.

Typically, the content of cryoprotectants, lyoprotectants and bulking agents need to be proper adjusted so as to contribute to the stability of Modified Factor VII proteins during freeze-drying and after termination of freeze-drying, when stored at various conditions.

The present investigators have found that the sugar alcohol should be in an amount ranging from about 30% w/w to 95% w/w. Preferably, the amount of the sugar alcohol should range from about 35% w/w to 95% w/w, more preferably from about 40% w/w to 90% w/w and most preferably from about 45% w/w to 90% w/w.

As can be seen from Example 3, the present investigators have provided compositions with low content of degradation products upon termination of the freeze-drying process (less than 24 hours after termination of the freeze-drying process) by including a proper content of the mannitol corresponding to 40 mg/ml (concentration before freeze-drying). Thus, the compositions according to the invention are characterised by having a low initial content of oxidised forms and aggregates before being subjected to storage.

Typically, the compositions are stabilised upon termination of the freeze-drying such that less than 4% w/w of the Modified Factor VII is converted into its oxidised forms, less than 1% w/w is recovered as dimeric forms and higher-order polymeric forms are not detected using conventional analytical methods (such as, e.g., the methods described in Example 2 of the present specification.

Moreover and advantageously, the compositions of the invention are storage-stable, e.g. less than 5% w/w, such as 4%, 3%, or 2.5%, of the Modified Factor VII is converted into an oxidised form and less than 5% w/w, such as 4%, 3%, or 2.5% is converted into a dimeric form upon storage at 25 °C for 12 months in the dark.

The present investigators provide data herein indicating that further degradation of Modified Factor VII is minimal upon storage under ambient conditions. As can be seen from Example 4 the increase is such that less than 2% w/w of Modified Factor VII, such as about 1% w/w is recovered as oxidised forms of Modified Factor VII in addition to the initial content of said oxidised forms present upon termination freeze-drying. Moreover, less than 2% w/w, such as about 1% w/w of Modified Factor VII is additionally recovered as dimeric forms upon storage for 12 months at 25°C. Of great importance, no increase in the content of polymeric forms was monitored during said storage at 25°C for 12 months.

As mentioned, the degradation of Modified Factor VII may result in oxidation as well as aggregation. However, the oxidation pathway was found to be more sensitive to storage for which reason the oxidised forms of Modified Factor VII are useful as a stability indicating parameter.

Thus in some embodiments, the invention relates to a composition that is stable upon storage of the composition for 12 months at 25 °C, such that oxidised forms of said Modified Factor VII are in an amount of at the most about 4% w/w relative to the initial theoretical content of said Modified Factor VII. The initial theoretical content of said Modified Factor VII being the amount added to the composition upon preparation of the composition before the freeze-drying step. By proper optimisation of the content of cryoprotectants, lyoprotectants and bulking agents, the oxidised forms of said Modified Factor VII are in an amount of at the most about 3.5% w/w, more preferably of at the most about 3.0% w/w. In a further suitable embodiment thereof, the oxidised forms of said Modified Factor VII are in an amount of at the most about 2.5% w/w relative to the initial theoretical content of said Modified Factor VII.

As mentioned, the invention relates in part to limiting the degradation of Modified Factor VII from the time of manufacturing the solid composition of Modified Factor VII, e.g. after termination of freeze-drying until the time of use, e.g. at the time when the composition is to be administered by a patient. Therefore, according to the present invention, suitable compositions have a limited increase in the content of oxidised forms upon storage for long-term at ambient conditions. That In further embodiments, the composition is stable such that upon storage of the composition for 12 months at 25 °C, oxidised forms of said Modified Factor VII increase in content compared to their initial content by no more than about 2.0% w/w relative to the initial theoretical content of said Modified Factor VII. In further suitable embodiments thereof, the increase in content of oxidised forms is at the most about 1.5% w/w, at the most about 1.0% w/w or preferably of at the most about 0.5% w/w. The increase in content of oxidised forms expresses the amount of Modified Factor VII recovered as oxidised forms upon storage.

The compositions according to the invention are also suitable for long-term storage at 25°C and may even possess an appropriate stability at conditions causing accelerated degradation, such as at higher temperatures, e.g. at 45°C.

In further embodiments, the composition is stable such that upon storage of the composition for 18 months at 25 °C, oxidised forms of said Modified Factor VII are in an amount of at the most about 4.5% w/w relative to the initial theoretical content of said Modified Factor VII. Preferably, the amount of oxidised forms is at the most about 4.0 % w/w, more preferably of at the most about 3.5% w/w, even more preferably of at the most about 3.0% w/w most preferably of at the most about 3.5% w/w.

That is further to say that the composition is stable such that following storage of the composition for 18 months at 25 °C, oxidised forms of said Modified Factor VII increase in content compared to their initial content by no more than about 2.8% w/w relative to the initial theoretical content of said Modified Factor VII. In further embodiments thereof, the increase corresponds to an increase in the content of oxidised forms in an amount of at the most about 2.5% w/w and in more preferably embodiments of at the most about 2.0% w/w. In still further embodiments, the increase in content of oxidised forms is of at the most about 1.5% w/w, most preferably of at the most about 1.0% w/w, such as at the most about 0.5 % w/w.

Still further embodiments of the invention relate to those wherein the composition is stable such that upon storage of the composition for 32 months at 25 °C, oxidised forms of said Modified Factor VII are in an amount of at the most about 9.0% w/w relative to the initial theoretical content of said Modified Factor VII, preferably in an amount of at the most about 8.0 % w/w, even more preferably of at the most about 7.0% w/w, still more preferably of at the most about most 6% w/w, such as about 5% w/w, preferably of at the most about 4% w/w, most preferably of at the most about 3% w/w.

In still further embodiments, the composition is stable such that following storage of the composition for 32 months at 25 °C, oxidised forms of said Modified Factor VII increase in content compared to their initial content by no more than about 7.0% w/w relative to the initial theoretical content of said Modified Factor VII, preferably in an amount of at the most about 6.0% w/w, more preferably of at the most about 5.0% w/w, such as the most of about 4% w/w and 3% w/w, even more preferably of at the most about 2.0% w/w most preferably of at the most about 1.0% w/w.

At accelerated conditions, the composition still has superior stability. As such, in other embodiments, the invention relates to a composition that is stable such that upon storage of the composition for 8 weeks at 45 °C, oxidised forms of said Modified Factor VII are in an amount of at the most about 4% w/w relative to the initial theoretical content of said Modified Factor VII, preferably in an amount of at the most about 3.5% w/w, even more preferably of at the most about 3.0% w/w, most preferably of at the most about 2.5% w/w.

Moreover, the increase of oxidised forms following termination of the freeze-drying process is limited. Thus in yet other embodiments the composition is stable such that upon storage of the composition for 8 weeks at 45 °C, oxidised forms of said Modified Factor VII increase in content compared to their initial content by no more than about 2.0% w/w relative to the initial theoretical content of said Modified Factor VII, preferably in an amount of at the most about 1.5% w/w, even more preferably of at the most about 1.0% w/w, even more preferably of at the most about 0,8% w/w most preferably of at the most about 0.5% w/w of oxidised forms.

Better stability is achieved upon storage at colder conditions such as at 5°C. At this temperature, the compositions may be stored for an even longer period of time before the degradation of Modified Factor VII is unsuitable. As can be seen from Example 4, less than about 1.5% w/w of Modified Factor VII is degraded upon storage for 32 months.

Thus, other embodiments of the invention relate to compositions stable such that upon storage of the composition for 32 months at 5 °C, oxidised forms of said Modified Factor VII are in an amount of at the most about 5.0 %w/w relative to the initial theoretical content of said Modified Factor VII, preferably in an amount of at the most about 4.5% w/w, more preferably of at the most about 4.0 % w/w, even more preferably of at the most about 3.5 % w/w most preferably of at the most about 3.0% w/w, such as about 2.5% w/w.

Furthermore, in still other embodiments, the composition is stable such that following storage of the composition for 32 months at 5 °C, oxidised forms of said Modified Factor VII increase in content compared to their initial content by no more than about 2.5% w/w relative to the initial theoretical content of said Modified Factor VII, preferably in an amount of at the most about 2.0% w/w, more preferably of at the most about 1.5% w/w, even more preferably of at the most about 1.0% w/w, most preferably of at the most about 0.5% w/w.

As discussed above, the improved stability relates, in part, to selecting suitable cryoprotectants, lyoprotectants and bulking agents and to adjust their content. As stated at least one group of such excipients should be present in the composition of the invention in order to achieve the proper stability. However, as was found by the present investigators, the stability is even more favourable upon combining excipients with the above-mentioned properties. Therefore, suitably embodiments of the invention relates to compositions further comprising a saccharide. Saccharides of interest are di- and tri-saccharides but also some polysaccharides such that the saccharides may be selected from the group consisting of sucrose, dextrose, lactose, maltose, trehalose, cyclodextrins, maltodextrins and dextrans.

The present investigators have recognised the proper combination of the sugar alcohols and the saccharides as well as their content so as, at least in part, to achieve favourable stability.

Thus, in some embodiments of the invention, the saccharide is present in the composition in an amount ranging from about 1% w/w to 45% w/w. In further interesting embodiments thereof, the amount ranges from about 5% w/w to 40% w/w, more interestingly from about 5% w/w to 35% w/w and most suitably from about 10% w/w to 30% w/w.

Importantly, the ratio between the sugar alcohol and the saccharide needs to be properly adjusted. In some embodiments of the invention, said sugar alcohol is in a weight ratio relative to said saccharide ranging from about 100:1 to 1:20. In other embodiments thereof, said weight ratio is from about 50:1 to 1:10, more preferably from about 20:1 to 1:5. In other embodiments, the weight ratio relates to ranges from about 10:1 to 1:2, and from about 4:1 to 1:2. However, as was found out by the present investigators (see Example 5), the lyophilised cake collapsed upon incorporating higher amounts of the saccharides. As such, some embodiments relate to those wherein said sugar alcohol is in a weight ratio relative to said saccharide ranging from about 3:1 to 1:1, such as from about 3:1 to 3:2.

Moreover, the favourable stability relates, at least in part, to the content of said sugar alcohol and said saccharide relative to the content of said Modified Factor VII. Therefore, in further embodiments of the invention, the compositions comprising Modified Factor VII have a weight ratio of Modified Factor VII relative to the sum of said sugar alcohol and said saccharide ranging from about 1:200 to 1:5. Preferably, the weight ratio ranges from about 1:100 to 1:8 and,more preferably, from about 1:75 to 1:10. In other embodiments, the weight ratio ranges from about 1:60 to 1:15 such as from about 1:50 to 1:20.

In some embodiments of the invention, the sugar alcohol is mannitol and in still other embodiments, the saccharide is sucrose.

Thus, in those embodiments, the ratio between mannitol and sucrose is such that said mannitol is in a weight ratio relative to said sucrose ranging from about 10:1 to 1:10. More interesting, said ratio is from about 10:1 to 1:5, still more interesting from about 5:1 to 1:2. However, when the composition is provided as a lyophylised cake, very suitable embodiments relates to those wherein said mannitol is in a weight ratio relative to said saccharide ranging from about 5:1 to 1:1, most preferably from about 4:1 to 5:4, such as from about 3:1 to 3:2.

In further embodiments of the invention, said Modified Factor VII is in a weight ratio relative to the sum of said mannitol and said sucrose ranging from about 1:100 to 1:5, preferably from about 1:75 to 1:10, more preferably from about 1:60 to 1:15 most preferably from about 1:50 to 1:20.

As may be understood, the stabilised composition according to the present invention is in solid form; i.e. the composition has been subject to freeze-drying. However, this implies that the composition originally is provided as a liquid that is to be freeze-dried. For purposes, at least in part, of stabilising the liquid composition before removal of moisture and for purposes of stabilising the solid composition upon storage, the composition comprises an agent capable of keeping the pH at an optimum level for preventing degradation. As of great importance this pH range of 4-7 is also within the physiological range upon parental administration of the composition.

Optionally, the compositions may include an antioxidant. The term antioxidant encompasses any substance that prevents chemical oxidation of the Modified Factor VII. Thus, in some favourable embodiments of the invention, the compositions further comprise an antioxidant. Non-limiting examples of suitable antioxidants include ascorbic acid, cysteine, homocysteine, cystine, cystathionine, methionine, gluthatione, and peptides containing any one of cysteine, homocysteine, cystine, cystathionine, methionine and gluthatione, in particular peptides containing 2 to 5 amino acid residues wherein at least one of the residues is a cysteine, homocysteine, cystine, cystathionine, methionine or gluthatione residue. In some embodiments thereof, the antioxidant is methionine, in particular L-methionine. The antioxidant is included at a concentration of from about 0.01 to about 5.0 mg/ml, such as from about 0.1 to about 5.0 mg/ml, from about 0.1 to about 4.0 mg/ml, from about 0.1 to 3.0 mg/ml, from about 0.1 to about 2.0 mg/ml, or from about 0.5 to about 3.0 mg/ml mg/ml, from about 1.0 to about 2.7 mg/ml, such as about 2.5 mg/ml,.

The antioxidant contributes, at least in part, to stabilising the composition during storage from the time of providing the composition with a moisture content of the most 3%, e.g. from the termination of the freeze-drying process until use. As can be seen from Example 4, the increase in oxidised forms upon storing a composition comprising methionine for 32 months at 25°C is 0.9 %w/w.

The compositions may further be formulated by incorporating other pharmaceutically acceptable excipients so as to achieve compositions acceptable for parenteral administration, in particular intravenous administration. Methods for preparing compositions for parental administration will be known or apparent to those skilled in the art and are described in more detail in, for example, Remington: The Science and Practice of Pharmacy, 19th ed., Mack Publishing Company, Easton, PA (1995). The term "excipients" includes pharmaceutical acceptable reagents to provide good lyophilised cake properties (bulking agents) as well as provide lyoprotection and cryoprotection of the protein, maintenance of pH, maintenance of acceptable tonicity as well as proper conformation of the protein during storage so that substantial retention of biological activity and protein stability is maintained.

Thus, according to the invention, the compositions further comprise a tonicity modifier. Tonicity modifiers include, but are not limited to, amino acids; small peptides (e.g., having from 2 to 5 amino acid residues); neutral salts; mono- or disaccharides; polysaccharides; sugar alcohols, or a mixture of at least two of said modifiers. Examples of tonicity modifiers include, but are not limited to, sodium chloride, potassium chloride, sodium citrate, sucrose, glucose, glycylglycine, and mannitol. Normally, the modifiers are present at a concentration of from about 1 to about 500 mM; from about 1 to about 300 mM; from about 10 to about 200 m M; or from about 20 to about 150 mM, depending on the other ingredients present. Neutral salts such as, e.g., sodium chloride or potassium chloride may be used. By "neutral salt" is meant a salt that is neither an acid nor a base when dissolved in aqueous solution.

In one embodiment the tonicity modifier is selected from the group consisting of sodium acetate, sodium lactate, sodium chloride, potassium chloride and calcium chloride. It is also noted, that compositions may comprise much higher concentrations of the tonicity-modifying agent as long as the composition is made isotonic prior to use, for example bulk compositions need not to be isotonic with the physiological range. Compositions that are to be administered by injection or infusion should preferably be isotonic with serum before use (i.e., about 300 ± 50 milliosmol/kg).

Further stabilisation of the freeze-dried protein can be obtained by the addition of surfactants. Thus, in some embodiments of the invention, the compositions further comprising a surfactant, the surfactant being selected from the group consisting of polysorbates, e.g. Tween®, such as polysorbate 20 or 80; polyoxyethylene alkyl ethers or poloxamers, such as poloxamer 188 (e.g. Pluronic®) or poloxamer 407, (e.g., Lutrol®) and other ethylene/polypropylene block polymers or polyethyleneglycol (PEG) such as PEG8000. Typically, the surfactants are added in an amount of from about 0.005 to about 5 mg/ml, such as from about 0.01 to about 5.0 mg/ml, from about 0.005 to about 3.0 mg/ml, from about 0.01 to about 3.0 mg/ml, from about 0.01 to about 2.0 mg/ml, from about 0.01 to about 1.0 mg/ml, from about 0.01 to about 0.5 mg/ml, from about 0.05 to about 0.5 mg/ml, or from about 0.05 to about 0.25 mg/ml,
Preferred amounts are from 0.01 to 3 mg/ml, such as from about 0.01 to about 2.0 mg/ml, from about 0.01 to about 1.0 mg/ml, from about 0.01 to about 0.5 mg/ml, from about 0.05 to about 0.5 mg/ml, or from about 0.05 to about 0.25 mg/ml, such as about 0.1 mg/ml for Tween 20 and/or Tween 80, and from about 0.05 to 3.0 mg/ml, such as from about 0.05 to about 2.0 mg/ml, from about 0.05 to about 1.0 mg/ml, from about 0.05 to about 0.5 mg/ml, or from about 0.05 to about 0.25 mg/ml for Poloxamer 188.

In some embodiments of the invention, the composition further comprises other pharmaceutical excipients acting as, e.g., a bulking agent. That is to say that other bulking agents than mannitol may also be included in the compositions. In particular, bulking agents are included in compositions prepared by freeze-drying.

In one embodiment, the composition contains: Modified FVII, CaCl2, NaCl, Glycylglycine, Mannitol, and Tween 80, has a moisture content of at the most 3%, and has a pH in the range of 4.0 to 7.0 when the composition is dissolved in water.

In another embodiment, the composition contains: Modified FVII, CaCl2, NaCl, Glycylglycine, Mannitol, Sucrose, Methionine, and Tween 80, has a moisture content of at the most 3%, and has a pH in the range of 4.0 to 7.0 when the composition is dissolved in water.

In further embodiments, the composition contains:

| Compound | Formulation A | Formulation B | Formulation C |
|---|---|---|---|
| FFR-rFVIIa | 1.8 to 2.2 mg/ml | 1.8 to 2.2 mg/ml | 1.8 to 2.2 mg/ml |
| CaCl2 x2H2O | 1.3 to 1.7 mg/ml | 1.3 to 1.7 mg/ml | 9.3 to 1.7 mg/ml |
| NaCl | 2.7 to 3.1 mg/ml | 2.7 to 3.1 mg/ml | 2.7 to 3.1 mg/ml |
| Glycylglycine, | 1.1 to 1.5 mg/ml | 1.1 to 1.5 mg/ml | 1.1 to 1.5 mg/ml |
| Mannitol | 25 to 30 mg/ml | 35 to 45 mg/ml | 25 to 30 mg/ml |
| Sucrose | 20 to 5 mg/ml | - | 20 to 5 mg/ml |
| Methionine | - | - | 0.25 mg/ml |
| Tween 80 | 0.05 to 0.15 mg/ml | 0.05 to 0.15 mg/ml | 0.05 to 0.15 mg/ml |
| pH | 5.0 to 7.0 | 5.0 to 7.0 | 5.0 to 7.0 |

In other embodiments, the composition contains:

| Compound | Formulation D | Formulation E | Formulation F |
|---|---|---|---|
| FFR-rFVIIa | 2.0 mg/ml | 2.0 mg/ml | 2.0 mg/ml |
| CaCl2 x2H2O | 1.47 mg/ml | 1.47 mg/ml | 1.47 mg/ml |
| NaCl | 2.92 mg/ml | 2.92 mg/ml | 2.92 mg/ml |
| Glycylglycine | 1.32 mg/ml | 1.32 mg/ml | 1.32 mg/ml |
| Mannitol | 26.7 mg/ml | 40 mg/ml | 26.7 mg/ml |
| Sucrose | 13.3 mg/ml | - | 13.3 mg/ml |
| Methionine | - | - | 0.25 mg/ml |
| Tween 80 | 0.1 mg/ml | 0.1 mg/ml | 0.1 mg/ml |
| pH | 6.0 | 6.0 | 6.0 |

As discussed above, the present investigators have provided a method for stabilising Modified Factor VII proteins by providing the proteins in solid compositions comprising selected pharmaceutically acceptable excipients, of which it is important to include an agent capable of keeping the pH in the range from 4 to 7.

Therefore, a further aspect of the invention relates to a method for preparing a stable Modified Factor VII. The method comprises the steps of:
i) providing said Modified Factor VII in a solution with pH in the range of 4 to 7.
ii) processing said solution so as to obtain a solid composition with a moisture content of at the most 3 % w/w.

In further embodiments thereof, the method comprises that said solution further comprises a sugar alcohol selected from the group consisting of mannitol, sorbitol and xylitol. In still further interesting embodiments, said solution further comprises a saccharide selected from the group consisting of sucrose, dextrose, lactose, maltose, trehalose, cyclodextrins, maltodextrins and dextrans. In preferable embodiments the sugar alcohol is mannitol and the saccharide is sucrose.

Preferably the content of the sugar alcohol and optionally the content of the saccharide in said solution i) should be adjusted so as to achieve superior stabilised Modified Factor VII proteins. According to the invention, the sugar alcohol should be in an amount ranging from about 1 mg/ml to 60 mg/ml, preferably from about 10 mg/ml to 50 mg/ml, more preferably from about 15 mg/ml to 45 mg/ml, most preferably from about 20 mg/ml to 40 mg/ml. The saccharide, if present, should be provided in an amount ranging from about 1 mg/ml to 50 mg/ml, preferably from about 2 mg/ml to 35 mg/ml, more preferably from about 5 mg/ml to 25 mg/ml, most preferably from about 10 mg/ml to 20 mg/ml.

Moreover, the ratio between the sugar alcohol and the saccharide should be adjusted. Suitable embodiments relates to wherein the sugar alcohol is in a weight ratio relative to said saccharide ranging from about 100:1 to 1:10, preferably from about 50:1 to 1:5, more preferably from about 20:1 to 1:3, even more preferably from about 10:1 to 1:2, still more preferably from about 4:1 to 1:2, such as still more preferably from about 3:1 to 1:1, most preferably from about 3:1 to 3:2.

In a preferred embodiment, the method for preparing a stable Modified Factor VII comprises freeze-drying. The freeze-drying relates to a process, wherein the solution comprising said Modified Factor VII is filled into lyophilisation vials or the like. Said Modified Factor VII may optionally be subjected to sterile filtration before start of freeze-drying. Cooling is put on the shelves of the freeze-drier in order to freeze the vials and the solution below critical product temperatures. Water is removed by introducing vacuum and condensation of water vapour on the ice-condenser of the freeze-drier. When the product is dry, usually less than 1% residual moisture content, (e.g., measured by Karl Fischer coulometric titration as described above) the vials are closed and capped. Manufacturing is finalised and the composition is now in a form of a lyophilised cake.

Such compositions, when administered to a patient by injectable means, need to be reconstituted in a suitable liquid before use. They may also be reconstituted for other purposes, e.g. for reformulation into other pharmaceutical compositions. However, the present invention does not preclude that the compositions may be administered to a patient in their solid form.

The compositions are reconstituted using an acceptable, preferably sterile, diluent or carrier, preferably an aqueous carrier. A variety of aqueous carriers may be used, e.g., water (e.g. Water For Injection/WFI), buffered water, saline (e.g. 0.4% saline), glycine (e.g. 0.3% glycine), and the like. The reconstitution diluent may also contain one or more salts, such as a calcium salt (e.g. CaCl2) or a combination of a sodium and a calcium salt (e.g. NaCl and CaCl2).

Thus, reconstituting a composition according to the invention in sterile Ringer's solution could make up a typical pharmaceutical composition for intravenous infusion.

The reconstituted compositions are intended for parental administration for prophylactic and/or therapeutic treatment. Preferably, the pharmaceutical compositions are administered parentally, i.e., intravenously, subcutanously, or intramuscularly, or they are administered by way of continuous or pulsative infusion.

Therefore, a still further aspect of the invention relates to the use of the solid stabilised composition for the preparation of a medicament for therapeutic treatment such as preventing blood clotting or preventing tissue factor mediated reactions.

That is to say that one aspect of the invention relates to the use of a Modified Factor VII for the preparation of a medicament for preventing blood clotting and/or preventing tissue factor mediated reactions, said medicament comprising a composition comprising;
i) Modified Factor VII;
ii) an agent suitable for keeping the pH of said composition in the range of 4 to 7 when said composition is dissolved in water; and
iii) a moisture content of at the most 3%.

Furthermore, in another aspect the invention relates to administering said stabilised Modified Factor VII to a patient for preventing blood clotting and/or preventing tissue factor mediated reactions. Thus, the invention relates to a method of preventing blood clotting and/or preventing tissue factor mediated reactions, comprising administering to a subject in need thereof, an effective amount of a composition comprising;
i) Modified Factor VII;
ii) an agent suitable for keeping the pH of said composition in the range of 4 to 7 when said composition is dissolved in water; and
iii) a moisture content of at the most 3%.

In different embodiments, the blood clotting is associated with a condition selected from the group consisting of: angioplasty, deep vein thrombosis, pulmonary embolism, stroke, disseminated intravascular coagulation (DIC), fibrin deposition in lungs and kidneys associated with gram-negative endotoxemia, and myocardial infarction.

In still other embodiments the tissue factor mediated reactions are associated with a condition selected from the group consisting of SIRS, ARDS, MOF, HUS, and TTP.

As stated the composition is in solid form. Accordingly, in a suitable embodiment the medicament should be suitable for being dissolved, which allows for parental administration of the medicament. Thus, when administering the compositions to a patient it comprises the step of dissolving the composition in a suitable liquid prior to the administering step.

Abbreviations
- FVII: Coagulation factor VII in its single chain form
- FVIIa: Coagulation factor VII in its cleaved, activated two-chain form
- rFVII (rFVIIa): Recombinant factor VII (recombinant factor VIIa)
- FVIIai: Modified Factor VII which is coagulation factor VII having at least one modification in its catalytic centre, which modification substantially inhibits the ability of the Modified Factor VII to activate plasma Factor X or IX.
- RFVIIai: Recombinant Modified Factor VIIa (recombinant FVIIai)
- Dansyl-EGR- FVIIa: Coagulation factor FVlla (FVII) wherein the catalytic centre has been inactivated by chemically reacting FVlla (FVII) with Dansyl-Glu-Gly-Arg chloromethylketone
- (dansyl-EGR- FVII): (dansyl-EGR-cmk)
- FFR-FVlla: Coagulation factor FVIIa (FVII) wherein the catalytic centre has been inactivated by
- (FFR-FVII): chemically reacting FVlla (FVII) with Phe-Phe-Arg chloromethylketone (FFR-cmk)
- PPA-FVIIa: FVIIa (FVII) reacted with D-Phe-Pro-Arg chloromethylketone (PPA-cmk)
- (PPA-FVII) Dansyl-EGR- cmk: Dansyl-Glu-Gly-Arg chloromethylketone
- FFR-cmk: D-Phe-Phe-Arg or Phe-Phe-Arg chloromethylketone
- PPACK: D-Phe-Pro-Arg chloromethylketone (PPA-cmk)
- DFFR-cmk: Dansyl-D-Phe-Phe-Arg or dansyl-Phe-Phe-Arg chloromethylketone
- Ser344-FVIIa: Coagulation factor FVIIa (FVII) wherein the catalytic centre has been inactivated by
- (Ser344-FVII): replacing the native amino acid in position 344 with serine.

### Assays:

### Factor VII biological activity

The biological activity of factor VIIa in blood clotting derives from its ability to (i) bind to tissue factor (TF) and (ii) catalyze the proteolytic cleavage of factor IX or factor X to produce activated factor IX or X (factor IXa or Xa, respectively).

Biological activity of factor VII polypeptides ("factor VII biological activity") may be quantified by measuring the ability of a preparation to promote blood clotting using factor VII-deficient plasma and thromboplastin (described,, e.g., in U.S. Patent No. 5,997,864). Alternatively, factor VIIa biological activity may be quantified by (i) Measuring the ability of factor VIIa or a factor VIIa -related polypeptide to produce activated factor X (factor Xa) in a system comprising TF embedded in a lipid membrane and factor X. (Persson et al., J. Biol. Chem. 272:19919-19924, 1997); (ii) Measuring factor X hydrolysis in an aqueous system; (iii) Measuring the physical binding of factor VIIa or a factor Vlla - related polypeptide to TF using an instrument based on surface plasmon resonance (Persson, FEBS Letts. 413:359-363, 1997); (iv) Measuring hydrolysis of a synthetic substrate by factor VIIa and/or a factor VIIa -related polypeptide; and (v) Measuring generation of thrombin in a TF-independent in vitro system.

### Biological activity of Modified Factor VII:

The biological activity of Modified Factor VII may be measured, e.g., by a competition clot assay essentially as described in WO 92/15686 (Example III) (Assay 1) or in one or more of the Assays 2 to 7 below.

Inhibition of FVIIa/phosphotipids-embedded TF-catalyzed activation of FX by Modified Factor VII - FXa generation assay (Assay 2):
In the following example all concentrations are final. Lipidated TF (10 pM), FVIIa (100 pM) and Modified Factor VII (0 - 50 nM) in HBS/BSA (50 mM hepes, pH 7.4, 150 mM NaCl, 5 mM CaCl2,1 mg/ml BSA) are incubated 60 min at room temperature before FX (50 nM) is added. The reaction is stopped after another 10 min by addition of ½ volume stopping buffer (50 mM Hepes, pH 7.4, 100 mM NaCl, 20 mM EDTA). The amount of FXa generated is determined by adding substrate S2765 (0.6 mM, Chromogenix, and measuring absorbance at 405 nm continuously for 10 min. IC50 values for TF antagonist inhibition of FVIIa/lipidated TF-mediated activation of FX may be calculated. The IC50 value for FFR-rFVIIa is 51 +/- 26 pM in this assay.

### Inhibition of 125I-FVIIa binding to cell surface TF by Modified Factor VII -

### TF binding assay (Assay 3):

In the following example all concentrations are final. Binding studies are employed using the human bladder carcinoma cell line J82 (ATTC No. HTB-1) or the human keratinocyte cell line (CCD1102KerTr ATCC No CRL-2310) or NHEK P166 (Clonetics No. CC-2507) all constitutively expressing TF. Confluent monolayers in 24-well tissue culture plates are washed once with buffer A (10 mM Hepes, pH 7.45,150 mM NaCl, 4 mM KCl, and 11 mM glucose) supplemented with 5 mM EDTA and then once with buffer A and once with buffer B (buffer A supplemented with with 1 mg/ml BSA and 5 mM Ca2+). The monolayers are preincubated 2 min with 100 µl cold buffer B. Varying concentrations of Modified Factor VII and radiolabelled FVIIa (0.5 nM 125I-FVIIa) are simultaneously added to the cells (final volume 200 µl). The plates are incubated for 2 hours at 4 °C. At the end of the incubation, the unbound material is removed, the cells are washed 4 times with ice-cold buffer B and lysed with 300 µl lysis buffer (200 mM NaOH, 1 % SDS and 10 mM EDTA). Radioactivity is measured in a gamma counter (Cobra, Packard Instruments). The binding data are analyzed and curve fitted using GraFit4 (Erithacus Software, Ltd., (U.K.). The IC50 value for FFR-rFVIIa is 4 nM in this assay.

### inhibition of FVIIa/sTF amidolytic activity (Assay 4):

Inhibition of FVlla-TF catalyzed amidolytic activity by Modified Factor VII is tested employing soluble human TF (10 nM), recombinant human FVlla (10 nM) and increasing concentrations of Modified Factor VII. Varying concentrations of the Modified Factor VII are preincubated with 10 nM sTF and 10 nM FVIIa in BSA buffer (50 mM Hepes, pH 7.4, 100 mM NaCl, 5 mM CaCl2 and 1 mg/ml BSA) for 60 min at room temperature before addition of substrate S2288 (1.2 mM, Chromogenix). The colour development is measured continuously for 30 min at 405 nm. Amidolytic activity is presented as mOD/min. IC50 values for inhibition of FVIIa/TF amidolytic activity by the modified factor VII may be calculated.

### Inhibition of FXa generation (Assay 5).

Lipidated TF (10 pM), FVlla (100 pM) and Modified Factor VII (0 - 50 nM) in BSA buffer (see assay 4) are incubated 60 min at room temperature before FX (50 nM) is added. The reaction is stopped after another 10 min by addition of ½ volume stopping buffer (50 mM Hepes, pH 7.4, 100 mM NaCl, 20 mM EDTA). The amount of FXa generated is determined by adding substrate S2765 (0.6 mM, Chromogenix, and measuring absorbance at 405 nm continuously for 10 min. IC50 values for modified factor VII-inhibition of FVlla/lipidated TF-mediated activation of FX may be calculated.

### TF-dependent clotting assay (Assay 6):

The assay is carried out on an ACL300 Research clotting apparatus (ILS Laboratories). Dilutions of Modified Factor VII in 50 mM imidazole, pH 7.4, 100 mM NaCl, 0.1 % BSA are mixed with 25 mM CaCl2 in the ratio of 2 to 5 and added to sample cups in the clotting apparatus. Thromboplastin from human, rat, rabbit, baboon, or pig diluted with the imidazole buffer to give clotting time of approximately 30 sec in samples without modified factor Vii is placed in reagent reservoir 2, and human, rat, rabbit, baboon, or pig plasma, in reagent reservoir 3. During the analysis 70 µl of the modified factor VII and CaCl2 mixture is transferred to 25 µl thromboplastin reagent and preincubated 900 sec before addition of 60 µl plasma and measuring of the clotting time. Maximal clotting time is set to 400 sec. A dilution of the thromboplastin is used as standard curve for converting clotting times into TF activity relative to the control without modified FVII added.

Inhibition of FVIIa/cell surface TF catalyzed activation of FX by Modified Factor VII (Assay 7): Monolayers of cells expressing human TF, e.g. human lung fibroblasts WI-38 (ATTC No. CCL-75), human bladder carcinoma cell line J82 (ATTC No. HTB-1), human keratinocyte cell line CCD 1102KerTr (ATCC no. CRL-231 0), human glioblastoma cell line U87, or human breast cancer cell line MDA-MB231, are employed as TF source in FVIIa/TF catalyzed activation of FX. Confluent cell monolayers in a 24-, 48- or 96-well plate are washed one time in buffer A (10 mM Hepes, pH 7.45, 150 mM NaCl, 4 mM KCI, and 11 mM glucose) and one time in buffer B (buffer A supplemented with 1 mg/ml BSA and 5 mM Ca2+). FVIIa (1 nM), FX (135 nM) and varying concentrations of modified factor VII in buffer B are simultaneously added to the cells. Alternatively the cells are preincubated 15 min with modified factor VII before addition of rFVIIa and FX. FXa formation is allowed for 15 min at 37°C. 50-µl aliquots are removed from each well and added to 50 µl stopping buffer (Buffer A supplemented with 10 mM EDTA and 1 mg/ml BSA). The amount of FXa generated is determined by transferring 50 µl of the above mixture to a microtiter plate well and adding 25 µl Chromozym X (final concentration 0.6 mM) to the wells. The absorbance at 405 nm is measured continuously and the initial rates of colour development are converted to FXa concentrations using a FXa standard curve.

The following examples are offered by way of illustration, not by way of limitation.

### EXAMPLES

### Example 1

Typical compositions are shown. Table 1 shows the concentration of active ingredient and excipients in the event where the composition is in liquid form, i.e. composition before freeze-drying or in the reconstituted solution after freeze-drying. Table 2 shows the concentration of active ingredient and excipients in the event where the composition is in solid form, i.e. in freeze-dried form. Compositions 04 and 05 are comparative compositions.

**Table 1. Compositions, content of excipients in solution.**

| Main Function: | Excipients: | Compositions Content mg/ml (mmol/l) | | |
|---|---|---|---|---|
| | | 05 | 04 | 107 |
| Active Ingredient | FFR-FVII or FRR-FVlla | 2 | 2 | 2 |
| Tonicity modifier | Sodium Chloride | 2.92 (50) | 2.92 (50) | 2.92 (50) |
| Tonicity modifier/ stabiliser | Calcium Chloride, 2H₂0 | 1.4 (10) | 1.47(10) | 1.47(10) |
| Buffering agent | Glycylglycine | 1.32 (10) | 1.32 (10) | 1.32 (10) |
| Surfactant | Tween | 0.1 | 0.1 | 0.1 |
| Bulking Agent/ Cryoprotectant/ Lyoprotectant | Mannitol | 26.7 (147) | 40 (220) | 26.7 (147) |
| Bulking Agent/ Cryoprotectant/ Lyoprotectant | Sucrose | 13.3 (39) | - | 13.3 (39) |
| Antioxidant | Methionine | - | - | 0.25 |
| | pH | 6 | 6 | 6 |

**Table 2. Compositions, content of excipients in solid form.**

| Function | Ingredients | Compositions Content (% w/w) | | |
|---|---|---|---|---|
| | | 05 | 04 | 107 |
| Active Ingredient | FFR-FVII or FRR-FVIIa | 4.2 | 4.2 | 4.2 |
| Tonicity modifier | Sodium Chloride | 6.1 | 6.1 | 6.1 |
| Tonicity modifier/ Stabiliser | Calcium Chloride, 2H₂0 | 3.1 | 3.1 | 3.1 |
| Buffering agent | Glycylglycine | 2.8 | 2.8 | 2.8 |
| Surfactant | Tween | 0.2 | 0.2 | 0.2 |
| Bulking Agent/ Cryoprotectant/ Lyoprotectant | Mannitol | 55.8 | 83.7 | 55.8 |
| Bulking Agent/ Cryoprotectant/ Lyoprotectant | Sucrose | 27.8 | | 27.8 |
| Antioxidant | Methionine | | | 0.5 |
| | pH | 6 | 6 | 6 |

### Example 2

Analytical methods used in determining stability indicating parameters:

### A. Determination of Oxidised forms by Reverse Phase HPLC (RP-HPLC):

HPLC Column: 4,5x250 mm column packed with butylbonded silica with a particle size of 5µm and pore size 300A. Column temperature: 70°C. Eluent A: water 99.9% v/v and trifluoracetic acid 0.1 % v/v. Eluent B: acetonitrile 80% v/v, trifluoracetic acid 0.09% v/v and water 19.91 % v/v*.* The column was eluted with a linear gradient from X% B to (X+13)% B in 30 minutes. Flow rate: 1.0 ml/min. Detection: 214 nm.

The oxidised forms are methionine sulfoxides of FFR-FVlla. The two main derivatives are Met(O)298-FFR-FVlla and Met(O)306-FFR-FVIIa.

The content of oxidised forms is expressed as the percentage of the initial amount of FFR-FVIIa in the composition that is recovered as oxidised forms of FFR-FVIIa. The initial amount of FFR-FVIIa relates to the amount of FFR-FVlla upon preparation of the composition before freeze-drying.

### B. Determination of polymers, oligomers or dimers of FFR-FVII by High Performance Gel Permeation Chromatography (GP-HPLC).

GP-HPLC was run on a Waters Protein Pak 300 SW column, 7.5x300 mm, using 0.2 M ammoniumsulfat pH 7.0 as the mobile phase. Flow rate: 0.5 ml/min and detection: 215 nm.

The content of aggregates is expressed as the percentage of the initial amount of FFR-FVlla in the composition that is recovered as dimeric and polymeric forms of FFR-FVlla. The initial amount of FFR-FVIIa relates to the amount of FFR-FVIIa upon preparation of the composition before freeze-drying.

### Example 3

The content of dimeric, polymeric and oxidised forms of FFR-VIIa after termination of freeze-drying is reported for the compositions of Example 1. The content expresses the amount of Modified Factor VII (here FFR-VIIa) that is recovered as dimers, polymers or oxidised forms.

**Table 3. Content of dimers, polymers and oxidised forms of FFR-VIIa**

| Stability indicating parameters | Compositions | | |
|---|---|---|---|
| | 05 | 04 | 107 |
| | mannitol-sucrose 2:1 | mannitol-sucrose 3:0 | mannitol-sucrose 2:1 + methionine |
| Dimers (% w/w) | 0.3 | 0.5 | 0.3 |
| Polymers (% w/w) | < 0.3 | < 0.3 | < 0.3 |
| Oxidised forms (% w/w) | 2.1 | 2.2 | 1.9 |

| | | | |
|---|---|---|---|
| < 0.3: quantification limit is 0.3. | | | |

### Example 4

The increase in the content of dimers, polymers and oxidised forms of FFR-VIIa during storage at 5 °C for 18 months, 25 °C for 6, 12, 18 and 32 months or at 45 °C for 8 weeks is reported for the compositions of Example 1. The storage time being calculated from the time of termination of freeze-drying. The increase in the content expresses the additional amount of FFR-VIIa that is recovered as a dimeric, a polymeric or a oxidised form upon said storage in that the composition typically comprises an initial amount of degradation products upon finalising the manufacture of the composition.

**Table 4. Storage at 5 °C for 18 months**

| Stability indicating parameters | Compositions | | |
|---|---|---|---|
| | 05 | 04 | 107 |
| | mannitol-sucrose 2:1 | mannitol-sucrose 3:0 | mannitol-sucrose 2:1 + methionine |
| Dimers | no increase | no increase | no increase |
| Polymers | no increase | no increase | no increase |
| Oxidised forms | no increase | no increase | no increase |

**Table 5. Storage at 25 °C for 6 months**

| Stability indicating parameters | Compositions | | |
|---|---|---|---|
| | 05 | 04 | 107 |
| | mannitol-sucrose 2:1 | mannitol-sucrose 3:0 | mannitol-sucrose 2:1 + methionine |
| Dimers | no increase | no increase | no increase |
| Polymers | no increase | no increase | no increase |
| Oxidised forms | 0.5 | 0.7 | no increase |

**Table 6. Storage at 25 °C for 12 months**

| Stability indicating parameters | Compositions | | |
|---|---|---|---|
| | 05 | 04 | 107 |
| | mannitol-sucrose 2:1 | mannitol-sucrose 3:0 | mannitol-sucrose 2:1 + methionine |
| Dimers | no increase | 0.7 | no increase |
| Polymers | no increase | no increase | no increase |
| Oxidised forms | 0.7 | 1.0 | no increase |

**Table 7. Storage at 25 °C for 18 months**

| Stability indicating parameters | Compositions | | |
|---|---|---|---|
| | 05 | 04 | 107 |
| | mannitol-sucrose 2:1 | mannitol-sucrose 3:0 | mannitol-sucrose 2:1 + methionine |
| Dimers | no increase | no increase | no increase |
| Polymers | no increase | no increase | no increase |
| Oxidised forms | 0.7 | 1.7 | 0.4 |

**Table 8. Storage at 25 °C for 32 months**

| Stability indicating parameters | Compositions | | |
|---|---|---|---|
| | 05 | 04 | 107 |
| | mannitol-sucrose 2:9 | mannitol-sucrose 3:0 | mannitol-sucrose 2:1 + methionine |
| Dimers | no increase | 0.6 | no increase |
| Polymers | no Increase | no increase | no increase |
| Oxidised forms | 2.0 | 6.1 | 0.9 |

**Table 9. Storage at 45 °C for 8 weeks**

| Stability indicating parameters | Compositions | | |
|---|---|---|---|
| | 05 | 04 | 107 |
| | mannitol-sucrose 2:1 | mannitol-sucrose 3:0 | mannitol-sucrose 2:1 + methionine |
| Dimers | 0.4 | 0.9 | no increase |
| Polymers | no increase | no increase | no increase |
| Oxidised forms | 0.8 | 0.9 | no increase |

### Example 5

Structural stability of the freeze-dried cake is shown for various compositions, 1-6. The content of mannitol and sucrose is reported of each composition (Table 10). The compositions comprise either FFR-FVII or FRR-FVIIa in concentration of 1 mg/ml and comprises in addition other excipients in an amount of 5.7 mg/ml, including, sodium chloride, calcium chloride 2H₂0, glycylglycine and tween. The structural stability of the freeze-dried cake is reported in table 11.

Compositions 1 and 4 are comparative compositions

**Table 10. Compositions comprising various amounts of mannitol and sucrose.**

| Content of Mannitol / Sucrose: | | Compositions | | | | | |
|---|---|---|---|---|---|---|---|
| | | 1 | 2 | 3 | 4 | 5 | 6 |
| Mannitol | mg/ml | 25 | 16.7 | 8.3 | 40 | 26.8 | 13.2 |
| | % w/w | 79 | 53 | 26 | 86 | 57 | 28 |
| | mM | 137 | 92 | 46 | 220 | 147 | 72 |
| Sucrose | mg/ml | - | 8.3 | 16.7 | - | 13.2 | 26.8 |
| | %w/w | - | 26 | 53 | - | 28 | 57 |
| | mM | - | 24 | 49 | - | 39 | 78 |

**Table 11. Stability of the freeze-dried cake according to the ratio between mannitol and sucrose**

| | Compositions | | | | | |
|---|---|---|---|---|---|---|
| | 1 | 2 | 3 | 4 | 5 | 6 |
| | | | | | | |
| Weight Ratio of Man : Suc | | 2:1 | 1:2 | | 2:1 | 1:2 |
| Molar Ratio of Man : Suc | | 4:1 | 9:10 | | 4:1 | 9:10 |
| Weight Ratio of Man + Suc : FFR-FVlla | 25:1 | 25:1 | 25:1 | 40:1 | 40:1 | 40:1 |
| Weight Ratio of Man: FFR-FVlla | 25:1 | 17:1 | 8:1 | 40:1 | 27:1 | 13:1 |
| Weight Ratio of Suc : FFR-FVIIa | | 8:1 | 17:1 | | 13:1 | 27:1 |
| Freeze-dried cake | OK | OK | C | OK | OK | C |
| Reconstituted solution | Clear sol. | Clear sol. | nd | Clear sol. | Clear sol. | nd |

| | | | | | | |
|---|---|---|---|---|---|---|
| C : collapsed cake, Man: Mannitol, Suc: Sucrose, Clear sol.: Clear solution, nd: not determined | | | | | | |

## Claims

1. A composition comprising;
i) Modified Factor VII;
ii) an agent suitable for keeping the pH of said composition in the range of 4 to 7 when said composition is dissolved in water;
iii) a sugar alcohol, and wherein the sugar alcohol is mannitol;
iv) a saccharide, and wherein the saccharide is sucrose;
v) an antioxidant, and wherein the antioxidant is selected from the group consisting of ascorbic acid, cysteine, homocysteine, cystine, cystathionine, methionine, gluthatione, and peptides containing any one of cysteine, homocysteine, cystine, cystathionine, methionine and gluthatione; and
vi) a moisture content of at the most 3%.

2. The composition according to any one of preceding claims, wherein the agent suitable for keeping the pH of said composition in the range of 4 to 7 is glycylglycine, then it is present in an amount of at the most 7 mg/ml.

3. The composition according to any one of preceding claims, wherein pH of said composition is in the range of about 5.5 to 7.0 when said composition is dissolved in water.

4. The composition according to any one of preceding claims, wherein said sugar alcohol is in a weight ratio relative to said saccharide ranging from about 100:1 1 to 1:50, preferably from about 50:1 to 1:10, more preferably from about 20:1 to 1:5, even more preferably from about 10:1 to 1:2, still more preferably from about 4:1 to 1:2, such as still more preferably from about 3:1 to 1:1, most preferably from about 3:1 to 3:2.

5. The composition according to any one of claims 1 to 3, wherein said Modified Factor VII is in a weight ratio relative to the sum of said sugar alcohol and said saccharide ranging from about 1:200 to 1:5, preferably from about 1:100 to 1:8, more preferably from about 1:75 to 1:10, even more preferably from about 1:60 to 1:15 most preferably from about 1:50 to 1:20.

6. The composition according to any one of preceding claims, wherein the sugar alcohol is in an amount ranging from about 30% w/w to 95% w/w, preferably from about 35% w/w to 95% w/w, more preferably from about 40% w/w to 90% w/w, most preferably from about 45% w/w to 90% w/w.

7. The composition according to any one of preceding claims, wherein the saccharide is in an amount ranging from about 1% w/w to 45% w/w, preferably from about 5% w/w to 40% w/w, more preferably from about 5% w/w to 35% w/w, most preferably from about 10% w/w to 30% w/w.

8. The composition according to any one of preceding claims, wherein said mannitol is in a weight ratio relative to said sucrose ranging from about 10:1 to 1:10, preferably from about 10:1 to 1:5, more preferably from about 5:1 to 1:2, even more preferably from about 5:1 to 1:1, most preferably from about 4:1 to 5:4, such as from about 3:1 to 3:2.

9. The composition according to any one of preceding claims, wherein said Modified Factor VII is in a weight ratio relative to the sum of said mannitol and said sucrose ranging from about ranging from about 1:100 to 1:5, preferably from about 1:75 to 1:10, more preferably from about 1:60 to 1:15 most preferably from about 1:50 to 1:20.

10. The composition according to any one of preceding claims, further comprising a tonicity modifier.

11. The composition according to claim 10, wherein the tonicity modifier is selected from the group consisting of sodium acetate, sodium lactate, sodium chloride, potassium chloride and calcium chloride.

12. The composition according to any one of preceding claims, further comprising a surfactant.

13. The composition according to claim 12, wherein the surfactant is selected from the group consisting of polysorbates, such as polysorbate 20 or 80; polyoxyethylene alkyl ethers or poloxamers, such as poloxamer 188 or 407.

14. A composition according any one of preceding claims, wherein the Modified Factor VII is selected from the list of: human and bovine factor VII, wherein the active site residue Ser344 is modified, replaced with Gly, Met, Thr, or more preferably, Ala; human factor VII, wherein the residue Lys341 is replaced; human factor VII, wherein the residue Asp242 is replaced; human factor VII, wherein the residue His193 is replaced; FVII-(K341A); FVII-(S344A); FVII-(D242A); FVII-(H193A); a factor VII polypeptide modified in the active site by reaction with a reagent selected from the list of: peptide chloromethylketones or peptidyl cloromethanes; azapeptides; acylating agents such as various guanidinobenzoate derivatives and 3-alkoxy-4-chloroisocoumarins; sulphonyl fluorides such as phenylmethylsulphonylfluoride (PMSF); diisopropylfluorophosphate (DFP); tosylpropylchloromethyl ketone (TPCK); tosylysylchloromethyl ketone (TLCK); nitrophenylsulphonates; heterocyclic protease inhibitors such as isocoumarines, and coumarins.

15. The composition according to any one of preceding claims, wherein the agent suitable for keeping the pH in the range of 4 to 7 is selected from the group consisting of citrate, histidine, malate, phosphate, tartaric acid, succinic acid, MES, HEPES, imidazol, TRIS, lactate, glutamate and glycylglycine, with the proviso that when said agent is glycylglycine, it is present in an amount of at the most 7 mg/ml.

16. The composition according to any one of preceding claims, wherein said content of moisture is at the most 2% w/w, most preferably at the most about 1% w/w of moisture.

17. The composition according to any one of preceding claims, wherein the composition is a lyophilised cake.

18. Use of a composition as defined in any one of the preceding claims for the preparation of a medicament.

## Patentansprüche

1. Zusammensetzung, umfassend:
i) modifizierten Faktor VII;
ii) ein Mittel, das beim Lösen der Zusammensetzung in Wasser zum Halten des pH-Werts der Zusammensetzung im Bereich von 4 bis 7 geeignet ist;
iii) einen Zuckeralkohol und wobei der Zuckeralkohol Mannit ist;
iv) ein Saccharid und wobei das Saccharid Saccharose ist;
v) ein Antioxidationsmittel und wobei das Antioxidationsmittel ausgewählt ist aus der Gruppe, bestehend aus Ascorbinsäure, Cystein, Homocystein, Cystin, Cystathionin, Methionin, Gluthation und Peptiden, die ein beliebiges von Cystein, Homocystein, Cystin, Cystathionin, Methionin, Gluthation enthalten; und
vi) einen Feuchtigkeitsgehalt von höchstens 3%.

2. Zusammensetzung nach einem der vorstehenden Ansprüche, wobei das zum Halten des pH-Werts im Bereich von 4 bis 7 geeignete Mittel Glycylglycin ist, wobei es dann in einer Menge von höchstens 7 mg/ml vorliegt.

3. Zusammensetzung nach einem der vorstehenden Ansprüche, wobei der pH-Wert der Zusammensetzung beim Lösen der Zusammensetzung in Wasser im Bereich von etwa 5,5 bis 7,0 liegt.

4. Zusammensetzung nach einem der vorstehenden Ansprüche, wobei der Zuckeralkohol in einem Gewichtsverhältnis in Bezug auf das Saccharid im Bereich von etwa 100:1 bis 1:50, vorzugsweise von etwa 50:1 bis 1:10, stärker bevorzugt von etwa 20:1 bis 1:5, noch stärker bevorzugt von etwa 10:1 bis 1:2, noch stärker bevorzugt von etwa 4:1 bis 1:2, wie noch stärker bevorzugt von etwa 3: bis 1:1, am meisten bevorzugt von etwa 3: bis 3:2 vorliegt.

5. Zusammensetzung nach einem der Ansprüche 1 bis 3, wobei der modifizierte Faktor VII in einem Gewichtsverhältnis in Bezug auf die Summe des Zuckeralkohols und des Saccharids im Bereich von etwa 1:200 bis 1:5, vorzugsweise von etwa 1:100 bis 1:8, stärker bevorzugt von etwa 1:75 bis 1:10, noch stärker bevorzugt von etwa 1:60 bis 1:15, am meisten bevorzugt von etwa 1:50 bis 1:20 vorliegt.

6. Zusammensetzung nach einem der vorstehenden Ansprüche, wobei der Zuckeralkohol in einer Menge im Bereich von etwa 30 Gew.-% bis 95 Gew.%, vorzugsweise von etwa 35 Gew.-% bis 95 Gew.-%, stärker bevorzugt von etwa 40 Gew.-% bis 90 Gew.-%, am meisten bevorzugt von etwa 45 Gew.-% bis 90 Gew.-% vorliegt.

7. Zusammensetzung nach einem der vorstehenden Ansprüche, wobei das Saccharid in einer Menge im Bereich von etwa 1 Gew.-% bis 45 Gew.%, vorzugsweise von etwa 5 Gew.-% bis 40 Gew.-%, stärker bevorzugt von etwa 5 Gew.-% bis 35 Gew.-%, am meisten bevorzugt von etwa 10 Gew.-% bis 30 Gew.-% vorliegt.

8. Zusammensetzung nach einem der vorstehenden Ansprüche, wobei das Mannit in einem Gewichtsverhältnis in Bezug auf die Saccharose im Bereich von etwa 10:1 bis 1:10, vorzugsweise von etwa 10:1 bis 1:5, stärker bevorzugt von etwa 5:1 bis 1:2, noch stärker bevorzugt von etwa 5:1 bis 1:1, am meisten bevorzugt von etwa 4:1 bis 5:4, wie von etwa 3:1 bis 3:2 vorliegt.

9. Zusammensetzung nach einem der vorstehenden Ansprüche, wobei der modifizierte Faktor VII in einem Gewichtsverhältnis in Bezug auf die Summe des Mannits und der Saccharose im Bereich von etwa 1:100 bis 1:5, vorzugsweise von etwa 1:75 bis 1:10, stärker bevorzugt von etwa 1:60 bis 1:15, am meisten bevorzugt von etwa 1:50 bis 1:20 vorliegt.

10. Zusammensetzung nach einem der vorstehenden Ansprüche, des Weiteren umfassend einen Tonizitätsmodifikator.

11. Zusammensetzung nach Anspruch 10, wobei der Tonizitätsmodifikator ausgewählt ist aus der Gruppe, bestehend aus Natriumacetat, Natriumlactat, Natriumchlorid, Kaliumchlorid und Calciumchlorid.

12. Zusammensetzung nach einem der vorstehenden Ansprüche, des Weiteren umfassend ein oberflächenaktives Mittel.

13. Zusammensetzung nach Anspruch 12, wobei das oberflächenaktive Mittel ausgewählt ist aus der Gruppe, bestehend aus Polysorbaten, wie Polysorbat 20 oder 80; Polyoxyethylenalkylethern oder Poloxameren, wie Poloxamer 188 oder 407.

14. Zusammensetzung nach einem der vorstehenden Ansprüche, wobei der modifizierte Faktor VII ausgewählt ist aus der Liste: Human- und Rinder-Faktor-VII, wobei der Rest der aktiven Stelle Ser₃₄₄ modifiziert ist indem er mit Gly, Met, Thr oder stärker bevorzugt Ala ersetzt ist; Human-Faktor-VII, wobei der Rest Lys341 ersetzt ist; Human-Faktor-VII, wobei der Rest Asp242 ersetzt ist, Human-Faktor-VII, wobei der Rest His193 ersetzt ist; FVII-(K341A); FVII-(S344A); FVII-(D242A); FVII-(H193A); ein Faktor-VII-Polypeptid, das an der aktiven Stelle durch Reaktion mit einem Reagenz modifiziert ist, das ausgewählt ist aus der Liste: Peptidchlormethylketone oder Peptidylchlormethane; Azapeptide, Acylierungsmittel wie verschiedene Guanidinbenzoatderivate und 3-Alkoxy-4-chlorisocumarine; Sulfonylfluoride wie Phenylmethylsulfonylfluorid (PMSF); Diisopropylfluorphosphat (DFP; Tosylpropylchlormethylketon (TPCK); Tosylysylchlormethylketon (TLCK); Nitrophenylsulfonate; heterocyclische Proteasehemmer wie Isocumarine und Cumarine.

15. Zusammensetzung nach einem der vorstehenden Ansprüche, wobei das zum Halten des pH-Werts im Bereich von 4 bis 7 geeignete Mittel ausgewählt ist aus der Gruppe, bestehend aus Citrat, Histidin, Malat, Phosphat, Weinsäure, Bernsteinsäure, MES, HEPES, Imidazol, TRIS, Lactat, Glutamat und Glycylglycin, mit der Maßgabe, dass, wenn das Mittel Glycylglycin ist, es in einer Menge von höchstens 7 mg/ml vorliegt.

16. Zusammensetzung nach einem der vorstehenden Ansprüche, wobei der Feuchtigkeitsgehalt höchstens 2 Gew.-%, am meisten bevorzugt höchstens etwa 1 Gew.-% Feuchtigkeit beträgt.

17. Zusammensetzung nach einem der vorstehenden Ansprüche, wobei die Zusammensetzung ein lyophilisierter Kuchen ist.

18. Verwendung einer wie in einem der vorstehenden Ansprüche definierten Zusammensetzung zur Herstellung eines Medikaments.

## Revendications

1. Composition comprenant :
i) un facteur VII modifié ;
ii) un agent permettant de maintenir le pH de ladite composition dans la plage de 4 à 7 quand ladite composition est dissoute dans l'eau ;
iii) un alcool de sucre, l'alcool de sucre étant le mannitol ;
iv) un saccharide, le saccharide étant le saccharose ;
v) un antioxydant, l'antioxydant étant choisi dans le groupe consistant en l'acide ascorbique, la cystéine, l'homocystéine, la cystine, la cystathionine, la méthionine, le glutathion, et les peptides contenant l'un quelconque de la cystéine, de l'homocystéine, de la cystine, de la cystathionine, de la méthionine et du glutathion ; et
vi) une teneur en humidité d'au plus 3 %.

2. Composition selon l'une quelconque des revendications précédentes, dans laquelle l'agent permettant de maintenir le pH de ladite composition dans la plage de 4 à 7 est la glycylglycine, laquelle est présente en une quantité d'au plus 7 mg/ml.

3. Composition selon l'une quelconque des revendications précédentes, le pH de ladite composition étant compris dans la plage d'environ 5,5 à 7,0 quand ladite composition est dissoute dans l'eau.

4. Composition selon l'une quelconque des revendications précédentes, dans laquelle ledit alcool de sucre est présent selon un rapport en poids avec ledit saccharide compris dans la plage d'environ 100:1 à 1:50, de préférence d'environ 50:1 à 1:10, d'une manière plus préférée d'environ 20:1 à 1:5, d'une manière encore plus préférée d'environ 10:1 à 1:2, d'une manière tout particulièrement préférée d'environ 4:1 à 1:2, notamment et tout spécialement d'environ 3:1 à 1:1, et d'une manière toute particulière d'environ 3:1 à 3:2.

5. Composition selon l'une quelconque des revendications 1 à 3, dans laquelle le facteur VII modifié présente un rapport en poids avec la somme dudit alcool de sucre et dudit saccharide compris dans la plage d'environ 1:200 à 1:5, de préférence d'environ 1:100 à 1:8, plus particulièrement d'environ 1:75 à 1:10, tout particulièrement d'environ 1:60 à 1:15, tout spécialement d'environ 1:50 à 1:20.

6. Composition selon l'une quelconque des revendications précédentes, dans laquelle l'alcool de sucre est présent en une quantité comprise dans la plage d'environ 30 à 95 % p/p, de préférence d'environ 35 à 95 % p/p, plus particulièrement d'environ 40 à 90 % p/p, tout spécialement d'environ 45 à 90 % p/p.

7. Composition selon l'une quelconque des revendications précédentes, dans laquelle le saccharide est présent en une quantité comprise dans la plage d'environ 1 à 45 % p/p, de préférence d'environ 5 à 40 % p/p, plus particulièrement d'environ 5 à 35 % en poids p/p, tout spécialement d'environ 10 à environ 30 % p/p.

8. Composition selon l'une quelconque des revendications précédentes, dans laquelle le mannitol est présent selon un rapport en poids avec ledit saccharose compris dans la plage d'environ 10:1 à 1:10, de préférence d'environ 10:1 à 1:5, plus particulièrement d'environ 5:1 à 1:2, tout particulièrement d'environ 5:1 à 1:1, tout spécialement d'environ 4:1 à 5:4, notamment d'environ 3:1 à 3:2.

9. Composition selon l'une quelconque des revendications précédentes, dans laquelle ledit facteur VII modifié est présent selon un rapport en poids avec la somme dudit mannitol et dudit saccharose compris dans la plage d'environ 1:100 à 1:5, de préférence d'environ 1:75 à 1:10, plus particulièrement d'environ 1:60 à 1:15, tout spécialement d'environ 1:50 à 1:20.

10. Composition selon l'une quelconque des revendications précédentes, comprenant en outre un agent modifiant la tonicité.

11. Composition selon la revendication 10, dans laquelle l'agent modifiant la tonicité est choisi dans le groupe consistant en l'acétate de sodium, le lactate de sodium, le chlorure de sodium, le chlorure de potassium et le chlorure de calcium.

12. Composition selon l'une quelconque des revendications précédentes, comprenant en outre un tensioactif.

13. Composition selon la revendication 12, dans laquelle le tensioactif est choisi dans le groupe consistant en les Polysorbates tels que le Polysorbate 20 ou 80, les alkyléthers polyéthoxylés ou les Poloxamers, tels que le Poloxamer 188 ou 407.

14. Composition selon l'une quelconque des revendications précédentes, dans laquelle le facteur VII modifié est choisi dans la liste comprenant le facteur VII humain ou bovin, dans lequel le résidu site actif Ser344 est modifié, remplacé par Gly, Met, Thr, ou plus particulièrement Ala ; le facteur VII humain, dans lequel le résidu Lys341 est remplacé ; le facteur VII humain, dans lequel le résidu Asp242 est remplacé ; le facteur VII humain dans lequel le résidu His193 est remplacé ; le FVII-(K341A) ; le FVII-(S344A) ; le FVII-(D242A) ; le FVII-(H193A) ; un polypeptide du facteur VII, modifié dans son site actif par réaction avec un réactif choisi dans la liste comprenant les chlorométhylcétones peptidiques ou les peptidylchlorométhanes, les azapeptides, les agents d'acylation tels que différents dérivés de guanidinobenzoate et les 3-alcoxy-4-chloroisocoumarines, les fluorures de sulfonyle tels que le fluorure de phénylméthylsulfonyle (PMSF), le fluorophosphate de diisopropyle (DFP), la tosylpropylchlorométhylcétone (TPCK), la tosyllysylchlorométhylcétone (TLCK), les nitrophénylsulfonates, les inhibiteurs de protéase hétérocycliques tels que les isocoumarines, et les coumarines.

15. Composition selon l'une quelconque des revendications précédentes, dans laquelle l'agent permettant de maintenir le pH dans la plage de 4 à 7 est choisi dans le groupe consistant en le citrate, l'histidine, le malate, le phosphate, l'acide tartrique, l'acide succinique, le MES, le HEPES, l'imidazole, le TRIS, le lactate, le glutamate et la glycylglycine, à la condition que, quand ledit agent est la glycylglycine, il soit présent en une quantité d'au plus 7 mg/ml.

16. Composition selon l'une quelconque des revendications précédentes, dans laquelle ladite teneur en humidité est d'au plus 2 % p/p, tout spécialement d'au plus environ 1 % p/p d'humidité.

17. Composition selon l'une quelconque des revendications précédentes, dans laquelle la composition est un gâteau lyophilisé.

18. Utilisation d'une composition telle que définie dans l'une quelconque des revendications précédentes pour la préparation d'un médicament.
